(19) Europäisches Patentamt
European Patent Office
Office européen des brevets

(11) Veröffentlichungsnummer: **0 007 533**
**B1**

(12) **EUROPÄISCHE PATENTSCHRIFT**

(45) Veröffentlichungstag der Patentschrift: **22.09.82**

(51) Int. Cl.³: **C 07 D 211/46,** C 08 K 5/34

(21) Anmeldenummer: **79102433.4**

(22) Anmeldetag: **13.07.79**

(54) **Piperidin-Derivate, Verfahren zu ihrer Herstellung und ihre Verwendung als Stabilisatoren für synthetische Polymere.**

(30) Priorität: **21.07.78 CH 7904/78**

(43) Veröffentlichungstag der Anmeldung:
**06.02.80 Patentblatt 80/3**

(45) Bekanntmachung des Hinweises auf die Patenterteilung:
**22.09.82 Patentblatt 82/38**

(84) Benannte Vertragsstaaten:
**BE CH DE FR GB IT**

(56) Entgegenhaltungen:
DE - A - 2 233 121
DE - A - 2 623 464
DE - A - 2 757 105
DE - A - 2 801 470
US - A - 3 940 363

Die Akte enthält technische Angaben, die nach dem Eingang der Anmeldung eingereicht wurden und die nicht in dieser Patentschrift enthalten sind.

(73) Patentinhaber: **CIBA-GEIGY AG**
**Patentabteilung Postfach**
**CH-4002 Basel (CH)**

(72) Erfinder: **Karrer, Friedrich, Dr.**
**Rebbergstrasse 5**
**CH-4800 Zofingen (CH)**

EP 0 007 533 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Piperidin-Derivate, Verfahren zu ihrer Herstellung und ihre Verwendung als Stabilisatoren für synthetische Polymere.

Die Erfindung betrifft neue 2,2,6,6-Tetramethylpiperidin-Derivate, sowie synthetische Polymere, enthaltend solche Derivate als Stabilisatoren.

Aus der US—A—3,940,363 sind Piperidin-Derivate als Stabilisatoren bekannt, so als Verbindung Nr. 1 das 1,2-Bis-(4-benzoyloxy-2,2,6,6-tetramethyl-piperidino)-äthan und als Verbindung Nr. 9 das 1,4-Bis-(4-benzyloxy-2,2,6,6-tetramethyl-piperidino)-trans-2-buten.

Es wurde nun gefunden, dass bestimmte 2,2,6,6-Tetramethylpiperidin-Derivate, in denen Verbindungen vom obigen Typ mit z.B. einem $\beta$-(3,5-Di-t-butyl-4-hydroxyphenyl)-propionyloxy-Rest in 4-Stellung des Piperidins verknüpft sind, besonders vorteilhaft sind. Solche Verbindungen zeigen neben einer überraschend verbesserten, Lichtschutzwirkung zugleich eine besonders gute Antioxidanswirkung in synthetischen Polymeren sowie eine gute Verarbeitungsstabilisierung in der Hitze. Die erfindungsgemässen Derivate sind schwer flüchtig, gut verträglich mit Polymeren und extraktionsstabil. Zudem haben sie hervorragende toxikologische Eigenschaften.

Die Erfindung betrifft neue 2,2,6,6-Tetramethylpiperidin-Derivate der Formel I

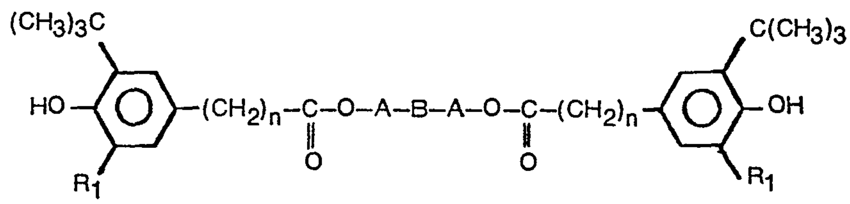

(I),

worin A ein Rest der Formel II B an das N-Atom gebunden ist,

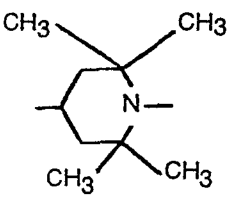

(II),

B $C_2$—$C_{12}$ Alkylen, $C_4$—$C_{10}$ Alkenylen, $C_6$ Alkinylen, Xylylen oder Bitolylen ist,
$R_1$ Wasserstoff oder $C_1$—$C_{12}$ Alkyl ist,
und
n 0,1 oder 2 ist.

B ist als $C_2$—$C_{12}$ Alkylen verzweigtes oder insbesondere geradkettiges Alkylen, vor allem mit 2—6 C-Atomen, wie Aethylen, 1,3-Propylen, 1,4-Butylen oder Hexamethylen, als $C_4$—$C_{10}$ Alkenylen verzweigtes oder insbesondere geradkettiges Alkenylen, vor allem mit 4—6 C-Atomen, wie 1,4-But-2-enylen oder 1,6-Hex-3-enylen, als $C_6$ Alkinylen insbesondere 1,6-Hex-3-inylen, und als Xylylen insbesondere p-Xylylen.

$R_1$ ist als $C_1$—$C_{12}$ Alkyl insbesondere solches mit 1—8 C-Atomen, wie Methyl oder tert.-Octyl, vor allem aber tert.-Butyl.

Bevorzugt sind Verbindungen der Formel I, worin B 1,4-But-2-enylen, p-Xylylen oder Bitolylen ist, $R_1$ Wasserstoff, Methyl oder tert.-Butyl ist, und n 0, 1 oder 2 ist, vor allem aber 2 ist.

Besonders bevorzugt sind die in den Beispielen genannten Verbindungen.

Die erfindungsgemässen Derivate können nach an sich bekannten Methoden hergestellt werden, z.B. wie in der US—A—3,940,363 in den Spalten 10 und folgende beschrieben ist, z.B. dadurch, dass man einen Niederalkylester der Formel V

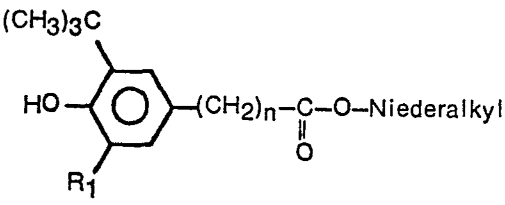

(V)

oder ein entsprechender Säurechlorid oder Säureanhydrid mit einer Verbindung der Formel HO—A—B—A—OH umsetzt.

Die Umesterung kann in an sich bekannter Weise erfolgen, insbesondere in einem inerten Lösungsmittel, wie einem Kohlenwasserstoff, z.B. Xylol oder Toluol, in Gegenwart eines Katalysators, wie einer starken Base z.B. Lithiumanid, Lithiummethylat, Aluminium-iso-propylat, Titan-n-butylat, Titan-iso-propylat, Natriummethylat oder Natriumäthylat. Falls erwünscht, kann ein Endprodukt mit einer Doppeloder Dreifachbindung, etwa mit B gleich 1,4-But-2-enylen, anschliessend hydriert werden, z.B. mit Wasserstoff in Gegenwart eines Edelmetallkatalysators oder Raney-Nickel, z.B. zu B gleich 1,4-Butylen. Die Umsetzung mit einem Säurechlorid erfolgt vorteilhaft in Gegenwart von mindestens 2 Moläquivalenten Base, wie einem tertiären Amin, z.B. Triäthylamin.

Die Ausgangsstoffe sind bekannt oder können, falls neu, nach an sich bekannten Methoden erhalten werden.

So kann man z.B. 4-Hydroxy-2,2,6,6-tetramethylpiperidin mit einem Hal-B-Hal umsetzen, worin Hal für Chlor oder Brom steht, vorzugsweise in Gegenwart einer Base und eines Lösungsmittels bei erhöhter Temperatur, wie etwa 50—160°. Geeignete Basen sind z.B. Alkalicarbonate, Alkali-hydroxyde oder Alkalioxyde. Geeignete Lösungsmittel sind z.B. Ketone, wie Aceton, Methyläthylketon, Diäthylketon, Methyl-isobutylketon oder Cyclohexanon, sowie Dimethylformamid, Dimethylsulfoxyd oder Sulfolan. Man erhält auf diese Weise ein Verbindung HO—A—B—A—OH mit A gleich Formel II, die dann gemäss oben weiter umgesetzt werden kann.

Die Verbindungen der Formel I können gemäss der vorliegenden Erfindung als Stabilisatoren für Kunststoffe gegen deren Schädigung durch Einwirkung von Sauerstoff, Wärme und Licht verwendet werden. Beispiele für solche Kunststoffe sind die in der DE—A 2,456,864 auf den Seiten 12—14 aufgeführten Polymeren.

Von besonderer Bedeutung ist die Stabilisierung von Polyolefinen, Styrolpolymerisaten und von Polyurethanen, für die sich die Piperidine der Formel I hervorragend eignen. Beispiele hierfür sind Polyäthylen hoher und niedriger Dichte, Polypropylen, Aethylen-Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von Lacken, Elastomeren oder Schaumstoffen.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Verbindungen können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Ausser den Verbindungen der Formel I können den Kunststoffen auch noch andere, bekannte Stabilisatoren zugesetzt werden. Dies können z.B. Antioxydantien, Lichtschutzmittel oder Metalldesaktivatoren sein, oder auch Costabilisatoren wie z.B. solche vom Typ der Phosphorigsäureester. Weiterhin können sonstige in der Kunststofftechnologie übliche Zusätze wie z.B. Flammschutzmittel, Antistatika, Weichmacher, Gleitmittel, Treibmittel, Pigmente, Verstärkungsstoffe oder Füllstoffe zugesetzt werden. Beispiele für Additive, die zusammen mit den Verbindungen der Formel I verwendet werden können, finden sich in der DE—A—2,427,853, Seiten 18—24.

Die Erfindung betrifft daher auch die durch Zusatz von 0,01—5 Gew.-% einer Verbindung der Formel I stabilisierten Kunststoffe, die gegebenenfalls noch andere bekannte und übliche Zusätze enthalten können. Die so stabilisierten Kunststoffe können in verschiedenster Form angewendet werden z.B. als Folien, Fasern, Bändchen, Profile oder als Bindemittel für Lacke, Klebemittel oder Kitte.

Die Herstellung und Verwendung der erfindungsgemässen Verbindungen wird in dem folgenden Beispiel näher beschrieben. Teile bedeuten darin Gewichtsteile und Prozent Gewichtsprozente. Die Temperaturen sind in Celsius-Graden angegeben.

Beispiel 1

Aus einer Lösung von 36,66 g 1,4-Bis-(2,2,6,6-tetramethyl-4-hydroxypiperidinyl-1-)-buten-2-(0,1 Mol, Smp. 208—210°), und 58,48 g 3-(3,5-Di-tert.-butyl-4-hydroxy)-phenyl-propionsäuremethylester (0,2 Mol) in 350 ml reinem Xylol (Isomerengemisch) wurden vorerst bei Normaldruck 50 ml Xylol durch den aufgesetzten, absteigenden Kühler ausdestilliert (azeotrope Entfernung der letzten Spuren Wasser). Hierauf versetzte man das Reaktionsgemisch mit 0,20 g Lithiumamid und erhitzte unter Rühren weiter, wobei das abgespaltene Methanol langsam, fortlaufend aus dem Reaktionsgemisch ausdestilliert wurde. Nach 2 Stunden war die Dampftemperatur von 135° erreicht (Badtemperatur 165°). Durch weitere, langsame Destillation wurd innerhalb von 15 Stunden das Xylol möglichst weitgehend

aus dem Reaktionsgemisch ausdestilliert. Im Dünnschichtchromatogramm waren nun keine Edukte mehr nachweisbar.

Zur Aufarbeitung löste man den auf 60° abgekühlten, viskosen Rückstand in 300 ml Chloroform, wusch viermal mit wenig Wasser, trocknete über Natriumsulfat, filtrierte und destillierte das Lösungsmittel im Vakuum (Rotationsverdampfer) vollständig ab. Das nach kurzer Zeit kristallin erstarrende Rohprodukte wurde zweimal in Methyläthylketon/Acetonitril (2:1) umkristallisiert, wodurch farblose, feine Kristalle von reinem 1,4 - Bis - {2,2,6,6 - tetramethyl - 4 - [3 - (3,5 - di - tert. - butyl - 4 - hydroxyphenyl)-propionyloxy]-piperidinyl-1}-buten-2 erhalten wurden. Die Verbindung weist einen Smp. von 152—153° auf.

Beispiele 2—9

In analoger Weise wie in Beispiel 1 beschrieben erhält man folgende Verbindungen:

Bis-{2,2,6,6-tetramethyl-4-[3-(3,5-di-tert.-butyl-4-hydroxyphenyl)-propionyloxy]-piperidinyl-1}-p-xylen (Beispiel 2), Smp. 213—214°.

Bis-{2,2,6,6-tetramethyl-4-[3-(3,5-di-tert.-butyl-4-hydroxyphenyl)-propionyloxy]-piperidinyl-1}-bitolylen (Beispiel 3), Smp. 134—135°.

1,4-Bis-{2,2,6,6-tetramethyl-4-(3,5-di-tert.butyl-4-hydroxybenzoyloxy)-piperidinyl-1}-buten-2 (Beispiel 4), Smp. 163—165°.

Bis-{2,2,6,6-tetramethyl-4-(3,5-di-tert.-butyl-4-hydroxybenzoyloxy)-piperidinyl-1}-p-xylylen (Beispiel 5), Smp. 157—160° und 250°—252° (2 Schmelzpunkte).

1,4-Bis-{2,2,6,6-tetramethyl-4-[2-(3,5-di-tert.-butyl-4-hydroxyphenyl)-acetyloxy]-piperidinyl-1}-buten-2 (Beispiel 6), Smp. 207—209°.

1,4-Bis-{2,2,6,6-tetramethyl-4-[3-(3-tert-butyl-4-hydroxy-5-methylphenyl)-propionyloxyl]-piperidinyl-1}-buten-2 (Beispiel 7), Smp. 157—158°.

Bis-{2,2,6,6-tetramethyl-4-[3-(3-tert.-butyl-4-hydroxyphenyl)-propionyloxy-piperidinyl-1}-p-xylylen (Beispiel 8), Smp. 182—185°.

1,4-Bis-{2,2,6,6-tetramethyl-4-[3-(3,5-di-tert.-butyl-4-hydroxyphenyl)-propionyloxyl]-piperidinyl-1}-buton (Beispiel 9), Smp. 162—164°.

**Patentansprüche**

1. 2,2,2,6-Tetramethylpiperidin-Derivate der Formel I

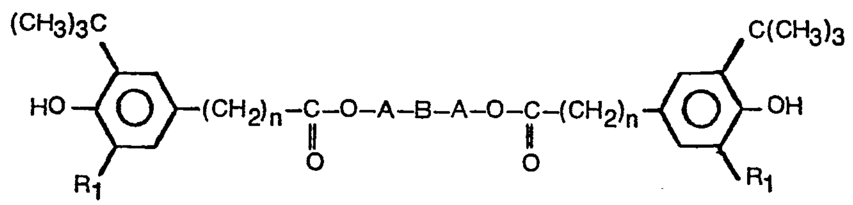

(I),

worin A ein Rest der Formel II ist und B an das N-Atom gebunden ist,

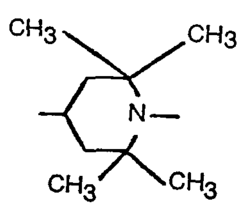

(II),

B $C_2$—$C_{12}$ Alkylen, $C_4$—$C_{10}$ Alkenylen, $C_6$ Alkinylen, Xylylen oder Bitolylen ist,
$R_1$ Wasserstoff oder $C_1$—$C_{12}$ Alkyl ist,
n 0, 1 oder 2 ist.

2. Verbindungen nach Anspruch 1, worin B 1,4-But-2-enylen, p-Xylylen oder Bitolylen ist, $R_1$ Wasserstoff, Methyl oder tert.-Butyl ist, und n 0, 1 oder 2 ist.

3. Verbindungen nach Anspruch 2, worin n 2 ist.

4. Verbindung nach Anspruch 1, das 1,4-Bis-{2,2,6,6-tetramethyl-4-[3,(3,5-di-tert.-butyl-4-hydroxyphenyl)-propionyloxy]-piperidinyl-1}-buten-2.

5. Stabilisiertes synthetisches Polymer, enthaltend als Stabilisator eine Verbindung gemäss einem der Ansprüche 1—4.

6. Verwendung eines Piperidins gemäss einem der Ansprüche 1—4 zum Stabilisieren von synthetischen organischen Polymeren.

7. Verfahren zur Herstellung von Verbindungen gemäss einem der Ansprüche 1—4, dadurch gekennzeichnet, dass man einen Niederalkylester der Formel V

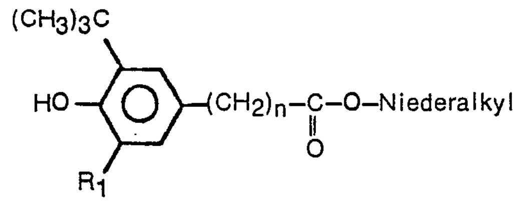

(IV)

oder ein entsprechendes Säurechlorid oder Säureanhydrid mit einer Verbindung der Formel HO—A—B—A—OH umsetzt, worin die Symbole die angegebenen Bedeutungen haben.

## Claims

1. A 2,2,6,6-tetramethylpiperidine derivative of the formula I

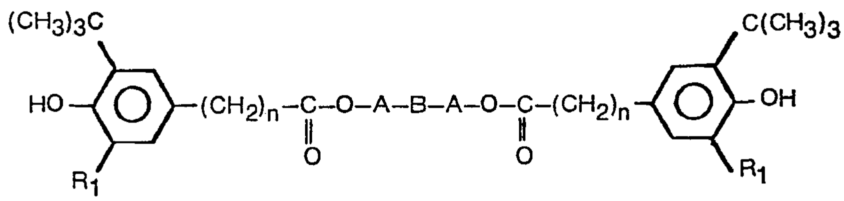

(I)

wherein A is a radical of the formula II and B is attached to the N atom,

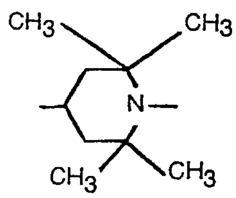

(II)

B is $C_2$—$C_{12}$ alkylene, $C_4$—$C_{10}$ alkenylene, $C_6$ alkynylene, xylylene or bitolylene, $R_1$ is hydrogen or $C_1$—$C_{12}$ alkyl, and n is 0, 1 or 2.

2. A compound according to claim 1, wherein B is 1,4-but-2-enylene, p-xylylene or bitolylene, $R_1$ is hydrogen, methyl or tert-butyl and n is 0, 1 or 2.

3. A compound according to claim 2, wherein n is 2.

4. A compound according to claim 1, which is 1,4-bis-{2,2,6,6-tetramethyl-4-[3,(3,5-di-tert-butyl-4-hydroxyphenyl)-propionyloxy]-piperidin-1-yl}-but-2-ene.

5. A stabilised synthetic polymer containing, as stabiliser, a compound according to any one of claims 1 to 4.

6. Use of a piperidine according to any one of claims 1—4 for stabilising a synthetic organic polymer.

7. A process for the preparation of a compound according to any one of claims 1 to 4, which comprises reacting a lower alkyl ester of the formula V

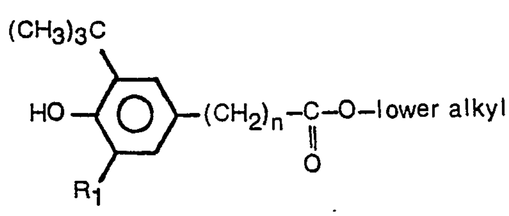

(V)

or a corresponding acid chloride or anhydride, with a compound of the formula HO—A—B—A—OH, in which formulae the symbols are as defined.

**Revendications**

1. Dérivés de la tétraméthyl-2,2,6,6 pipéridine répondant à la formule I

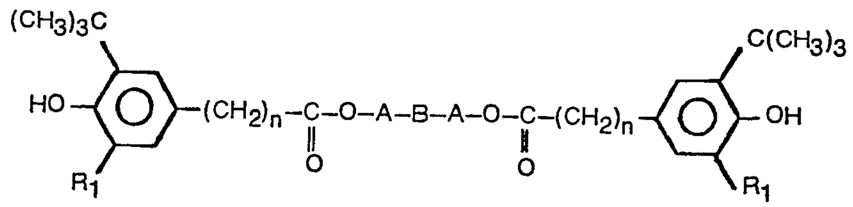

(I),

dans laquelle
A représente un radical de formule II qui est relié par son atome d'azote à B,

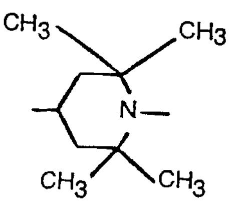

(II)

B représente un alkylène en $C_2$—$C_{12}$, un alcénylène en $C_4$—$C_{10}$, un alcynylène en $C_6$, un xylylène ou un bitolylène,
$R_1$ représente l'hydrogène ou un alkyle en $C_1$—$C_{12}$ et
n est égal à zéro, à 1 ou à 2.

2. Composés selon la revendication 1, dans lesquels B représente un radical butène-2 ylène-1,4, p-xylylène ou bitolylène, $R_1$ l'hydrogène, un méthyle ou un tert-butyle, et n est égal à zéro, à 1 ou à 2.

3. Composés selon la revendication 2 dans lesquels n est égal à 2.

4. Composé selon la revendication 1, en l'espèce le bis-{tétraméthyl-2,2,6,6[(di-tert-butyl-3,5 hydroxy-4 phényl)-3 propionyloxy]-4 pipéridinyl-1}-1,4 butène-2.

5. Polymère synthétique stabilisé qui contient, comme stabilisant, un composé selon l'une quelconque des revendications 1 à 4.

6. Application d'une pipéridine selon l'une quelconque des revendications 1 à 4 pour la stabilisation de polymères organiques synthétiques.

7. Procédé de préparation de composés selon l'une quelconque des revendications 1 à 4, caractérisé en ce qu'on fait réagir un ester alkylique inférieur de formule V

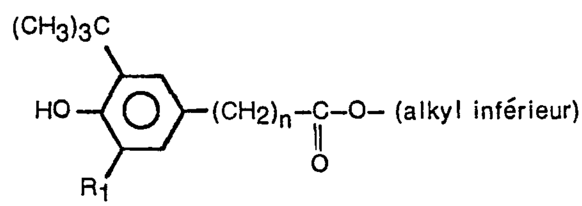

(V)

ou un chlorure d'acide ou anhydride d'acide correspondant, avec un composé de formule HO—A—B—A—OH dans lequel les symboles ont les significations précédemment données.